Europäisches Patentamt

European Patent Office

Office européen des brevets

(11) Publication number: **0 630 973 A2**

# EUROPEAN PATENT APPLICATION

(21) Application number: 94201349.1

(22) Date of filing: 11.05.94

(51) Int. Cl.⁵: **C12Q 1/68**, C12P 19/34, G01N 33/52, //C07H21/04, C12Q1/70

(30) Priority: 14.05.93 US 62023
14.05.93 US 62021
14.05.93 US 62022

(43) Date of publication of application:
28.12.94 Bulletin 94/52

(84) Designated Contracting States:
AT BE CH DE DK FR GB IT LI NL SE

(71) Applicant: EASTMAN KODAK COMPANY
343 State Street
Rochester,
New York 14650-2201 (US)

(72) Inventor: Cummins, Thomas J., c/o EASTMAN
KODAK CO.
Patent Department,
343 State Street
Rochester,
New York 14650-2201 (US)
Inventor: Atwood, Susan Melissa, c/o
EASTMAN KODAK CO.
Patent Department,
343 State Street
Rochester,
New York 14650-2201 (US)
Inventor: Bergmeyer, Lynn, c/o EASTMAN
KODAK CO.
Patent Department,

343 State Street
Rochester,
New York 14650-2201 (US)
Inventor: Findlay, John Bruce, c/o EASTMAN
KODAK CO.
Patent Department,
343 State Street
Rochester,
New York 14650-2201 (US)
Inventor: Sutherland, John W.H., c/o
EASTMAN KODAK CO.
Patent Department,
343 State Street
Rochester,
New York 14650-2201 (US)
Inventor: Kerschner, JoAnne H., c/o
EASTMAN KODAK CO.
Patent Department,
343 State Street
Rochester,
New York 14650-2201 (US)

(74) Representative: Aufflick, James Neil et al
Kodak Limited
Patent Department
Headstone Drive
Harrow
Middlesex HA1 4TY (GB)

(54) **Diagnostic compositions, elements, methods & test kits for amplification & detection of two or more DNA's using primers having matched melting temperatures.**

(57) An aqueous composition containing primers for opposing strands of two or more target nucleic acids can be used in polymerase chain reaction to provide simultaneously rapid and efficient amplification and detection of those nucleic acids. The primers for each target DNA differ in length by no more than 5 nucleotides and have a $T_m$ within the range of from 65 to 74°C, while the $T_m$'s are within about 5°C of each other. Such compositions are useful in diagnostic test kits and methods for amplification and detection of multiple nucleic acids, or in "multiplexing", using multiple capture probes. All of the capture probes have $T_m$'s which are greater than 50°C and are within 15°C of each other.

This invention relates to diagnostic compositions, elements, methods and test kits for the amplification and detection of a multiplicity of nucleic acids associated with one or more infectious agents. In particular, it relates to improved methods of polymerase chain reaction (PCR) using test kits and buffered compositions containing "matched" primers for a bacterial or viral DNA.

Technology to detect minute quantities of nucleic acids associated with various infectious agents (including viruses, bacteria, fungus and protozoa) has advanced rapidly over the last ten years including the development of highly sophisticated hybridization assays using probes in amplification techniques such as PCR. Researchers have readily recognized the value of such technology to detect diseases and genetic features in human or animal test specimens. The use of probes and primers in such technology is based upon the concept of complementarity, that is the bonding of two strands of a nucleic acid by hydrogen bonds between complementary nucleotides (also known as nucleotide pairs).

PCR is a significant advance in the art to allow detection of very small concentrations of a targeted nucleic acid. The details of PCR are described, for example, in US-A-4,683,195, US-A-4,683,202, and US-A-4,965,188 and by Mullis et al, *Methods of Enzymology*, 155, pp. 335-350 (1987). Without going into extensive detail, PCR involves hybridizing primers to the strands of a targeted nucleic acid (considered "templates") in the presence of a polymerization agent (such as a DNA polymerase) and deoxyribonucleoside triphosphates under the appropriate conditions. The result is the formation of primer extension products along the templates, the products having added thereto nucleotides which are complementary to the templates.

Once the primer extension products are denatured and one copy of each template has been prepared, the cycle of priming, extending and denaturation can be carried out as many times as desired to provide an exponential increase in the amount of nucleic acid which has the same sequence as the target nucleic acid. Despite the broad and rapid use of PCR in a variety of biological and diagnostic fields, there are still practical limitations which must be overcome to achieve the optimum success of the technology.

It is well known that PCR is susceptible to a "carry-over" problem whereby amplified nucleic acids from one reaction may be inadvertently carried over into subsequent reactions using "fresh" PCR reaction mixtures, and thereby causing "false" positives when testing later specimens.

One approach to this problem is to completely contain the reagents for each PCR procedure so no reagents or by-products can be carried over into later reactions. Specially designed test packs or test devices have been designed to contain PCR procedures for this reason. Such test packs are described, for example, in US-A-5,229,297. These test devices can be used in PCR in combination with automatic PCR processing equipment such as that described in US-A-5,089,660 and in US-A-5,089,233. This equipment is characterized by its capability to simultaneously process several test specimens in separate test devices.

More preferably, it would be desirable to detect a multiplicity of target nucleic acids (or a multiplicity of nucleic acid sequences in the same nucleic acid) in a single test device. This is referred to herein as "multiplexing".

In one embodiment of PCR, a specific set of primers and a capture probe (a total of three oligonucleotides) are needed for each target nucleic acid which is to be amplified and detected. Thus, the three oligonucleotides are complementary and specific to that target nucleic acid. For example, in multiplexing, if three target nucleic acids are to be amplified and detected, typically three sets of primers and probes are needed, one set specific for each target nucleic acid. Normally, detection of the multiple nucleic acids requires a multiplicity of test devices, and perhaps different sets of PCR conditions (that is, temperature and time conditions) to obtain efficient amplification of each target nucleic acid.

It would be desirable, however, to amplify and detect a plurality of target nucleic acids simultaneously in the same test device, using "universal" processing equipment and conditions. This cannot be done by merely selecting any set of primers and probes specific for each target nucleic acid from conventional sources. Where processing equipment is used to process several test devices simultaneously, or a single test device is designed for multiplexing, the equipment must be somehow adapted to provide optimum heating and cooling times and temperatures for each set of primers and probes, since they will all likely have individual optimum amplification conditions (for example, denaturation temperatures). To adapt the equipment to randomly selected primers and probes in multiplexing would be a very expensive and cumbersome solution to the problem. Yet there is a great need for efficient, relatively inexpensive and rapid multiplexing to detect multiple nucleic acids, or two or more nucleic acid sequences of the same nucleic acid.

The problems noted above are overcome by using, in PCR, an aqueous composition buffered to a pH of from 7 to 9, which comprises:

   a) first and second primers which are specific to and hybridizable with, respectively, first and second nucleic acid sequences which are in opposing strands of a first target DNA and which are separated from

2

each other along the opposing strands by from 90 to 400 nucleotides, and

b) third and fourth primers which are specific to and hybridizable with, respectively, third and fourth nucleic acid sequences which are in opposing strands of a second target DNA which is the same as or different from the first target DNA, the third and fourth nucleic acid sequences being different from the first and second nucleic acid sequences and being separated from each other along the opposing strands by from 90 to 400 nucleotides,

the composition characterized wherein each of the first, second, third and fourth primers has a $T_m$ - (defined below) within the range of from 65 to 74°C, all of the primer $T_m$'s being within 5°C of each other, the first and second primers have nucleotide lengths which differ from each other by no more than 5 nucleotides, and the third and fourth primers have nucleotide lengths which differ from each other by no more than 5 nucleotides.

This invention also provides a diagnostic test kit for the amplification of a first target DNA comprising, in separate packaging:

a) the aqueous composition as described above, and

b) at least one additional PCR reagent.

A method of this invention for the simultaneous amplification and detection of a first target DNA and a second target DNA comprises:

A) simultaneously subjecting the denatured opposing strands of a first target DNA and the denatured opposing strands of a second target DNA to polymerase chain reaction in the presence of:

i) the aqueous composition described above, and

ii) the additional PCR reagents: a thermostable DNA polymerase, a DNA polymerase cofactor and dNTP's, any or all of the additional PCR reagents being in the same or a different composition as defined in i),

to simultaneously amplify the opposing first target DNA strands and the opposing second target DNA strands,

B) simultaneously detecting at least one of the amplified first target DNA strands and at least one of the amplified second target DNA strands as a simultaneous determination of the presence of the first and second target DNA's.

Moreover, a diagnostic element comprises a water-insoluble, heat or ultrasonic sealable support, having disposed thereon in distinct regions thereof, a plurality of capture reagents,

the element characterized wherein each of the capture reagents has a capture probe specific for and hybridizable with a distinct target DNA associated with an infectious agent at a temperature of from 40 to 55°C, each of the capture probes has from 10 to 40 nucleotides and a $T_m$ greater than 50°C, and the $T_m$'s of all capture probes differ by no more than 15°C.

Novel oligonucleotides of this invention have one of the sequences:

```
SEQ ID:NO:26    5'-GAGATGGGAA TCCATATGCT GTATGTGAT-3',
SEQ ID:NO:27    5'-GGACACAGTG GCTTTTGACA GTTAATACA-3',
SEQ ID:NO:28    5'-GATGGTCCAG CTGGACAAGC AGAAC-3',
SEQ ID:NO:29    5'-CCTAGTGTGC CCATTAACAG GTCTTC-3',
SEQ ID:NO:30    5'-GACACAGAAA ATGCTAGTGC TTATGCAGC-3',
SEQ ID:NO:31    5'-GGTGGACAAT CACCTGGATT TACTGCAAC-3',
SEQ ID:NO:32    5'-CCTGATCTGT GCACGGAACT GAACACT-3',
SEQ ID:NO:33    5'-CCCAGTGTTA GTTAGTTTTT CCAATGTGTC TG-
                3',
SEQ ID:NO:34    5'-TGCCTGCGGT GCCAGAAACC GTTGAAT-3',
SEQ ID:NO:35    5'-TGCTCGGTTG CAGCACGAAT GGCACT-3',
SEQ ID:NO:36    5'-GAGCCGAACC ACAACGTCAC ACAATGTT-3',
SEQ ID:NO:37    5'-GGACACACAA AGGACAGGGT GTTCAGAAA-3',
SEQ ID:NO:39    5'-GCGACTCAGA GGAAGAAAC GATG-3',
SEQ ID:NO:40    5'-GAGATCGAGC TGGAGGATCC GTACG-3',
SEQ ID:NO:41    5'-AGCTGCAGCC CAAAGGTGTT GGACT-3',
SEQ ID:NO:51    5'-GGAACAACAT TAGAACAGCA ATACAACAAA CCG-
                3',
SEQ ID:NO:52    5'-AATATTGTAA CCTTTTGTTG CAAGTGTGAC TC-
                3',
SEQ ID:NO:53    5'-CCTATAGGTG GTTTGCAACC AATTAAACAC-3',
SEQ ID:NO:54    5'-GAGGTATTTG AATTTGCATT TAAAGATTTA
                         TTTGT-3',
SEQ ID:NO:55    5'-GCAAGACAGT ATTGGAACTT ACAGAGG-3',
SEQ ID:NO:56    5'-GTGTTGTAAG TGTGAAGCCA GATTTGA-3',
SEQ ID:NO:57    5'-GAGCAGATTG CGGCCACCGC AGCGATTTCG-3',
SEQ ID:NO:63    5'-CCGGGAGATG GGGGAGGCTA ACTGA-3',
SEQ ID:NO:64    5'-GGGGTGGGGA AAAGGAAGAA ACGCG-3', or
SEQ ID:NO:65    5'-AAAGACAGAA TAAAACGCAC GGGTGTTGGG TCG-
                3'.
```

The present invention provides an effective and efficient means for multiplexing, or amplifying and detecting a multiplicity of target nucleic acid sequences using the same test device, if desired, and the same processing equipment (processing one or more test devices simultaneously). It is particularly useful for the detection of one or more nucleic acid sequences of a first DNA associated with an infectious agent and one or more nucleic acid sequences of a second target DNA associated with the same or another infectious agent. Any number of nucleic acid sequences of the same or different DNA molecules can be amplified and determined simultaneously using the appropriate primer sets in combination.

These advantages are achieved by using a set of "matched" primers in PCR for each target nucleic acid. By "matched" primers is meant primers in each set having melting temperatures ($T_m$'s) which are essentially the same, that is they differ by no more than 5°C. Moreover, the $T_m$'s of the two primers of each set are within the range of from 65 to 74°C, and the two primers in each primer set have nucleotide lengths which differ from each other by no more than 5 nucleotides. Further, all of the primers of all primer

4

sets used in an amplification method are also "matched", that is, they all have $T_m$'s which differ by no more than 5°C and all are within the range of from 65 to 74°C.

FIGS. 1-6 are sets of bar graphs showing dye signals for replicate PCR assays of various concentrations of both of hCMV DNA and HIV-I DNA, as described in Example 2 below.

FIGS. 7 and 8 are sets of bar graphs showing dye signals for replicate PCR assays of various concentrations of HIV-I DNA, as described in Example 3 below.

FIGS. 9 and 10 are sets of bar graphs showing dye signals for replicated PCR assays of various concentrations of hCMV DNA, as described in Example 5 below.

The general principles and conditions for amplification and detection of nucleic acids using polymerase chain reaction are quite well known, the details of which are provided in numerous references including US-A-4,683,195, US-A-4,683,202 and US-A-4,965,188.

The present invention is directed to the amplification and detection of two or more specific nucleic acid sequences from DNA molecules associated with infectious agents in a test specimen. Such specimens can include bacterial or viral material, hair, body fluids or cellular materials containing DNA which can be detected.

Bacteria which can be detected include bacteria found in human blood, Salmonella species, Streptococcus species, Chlamydia species, Gonococcal species, *Mycobacterium tuberculosis, Mycobacterium fortuitum, Mycobacterium avium* complex, *Legionella pneumopbila, Clostridium difficile, Borrelia burgdorferei, Pneumocystis carinii, Mycoplasma Haemophilus influenzae*, Shigella species and Listeria species. Viruses which are detectable include cytomegalovirus, herpes, Epstein Barr virus, influenza viruses, human papilloma virus, hepatitis and retroviruses such as HTLV-I, HTLV-II, HIV-I and HIV-II. Protozoan parasites, yeasts and molds are also detectable. Other detectable species would be readily apparent to one skilled in the art.

The invention is particularly useful for the simultaneous amplification and detection of one or more nucleic acid sequences of a retroviral DNA (such as HTLV-I, HTLV-II, HIV-I or HIV-II DNA), human cytomegaloviral (hCMV) DNA, human papilloma viral DNA, *Mycobacterium tuberculosis* DNA, *Mycobacterium avium* DNA, hepatitis viral DNA and *Pneumocystis carinii* DNA.

A "target" DNA as used in this application also includes nucleic acids which are added to a test specimen to provide positive controls in an assay.

A "PCR reagent" refers to any of the reagents considered essential to PCR, namely primers for the target nucleic acid, a thermostable DNA polymerase, a DNA polymerase cofactor, and deoxyribonucleoside-5'-triphosphates.

The term "primer" refers to an oligonucleotide, whether naturally occurring or synthetically produced, which is capable of acting as a point of initiation of synthesis when placed under conditions in which synthesis of a primer extension product complementary to a nucleic acid strand (that is, template) is induced.

Generally, the primers used in this invention will have from 12 to 60 nucleotides, and preferably, they have from 20 to 40 nucleotides. More preferably, each primer in a set has from 25 to 35 nucleotides. The lengths of the primers in each primer set differ from each other by no more than 5 nucleotides, and preferably by no more than 2 nucleotides. Most preferably, the primers within a set have the same length.

One set of primers used in the practice of the invention includes first and second primers which are specific to, respectively, first and second nucleic acid sequences in opposing strands of a first target DNA. The first and second sequences are spaced along the opposing strands from each other by from 90 to 400 nucleotides, and preferably from 100 to 300 nucleotides apart on opposing strands. Thus, the two primers hybridize to nucleic acid sequences which are relatively close to each other along the opposing strands.

A second set of primers (including third and fourth primers) is used to amplify and detect third and fourth sequences of opposing strands of the same target DNA or of another target DNA from a different source. Additional sets of primers can be used to amplify and detect additional target DNA's.

For every set of primers used in this invention, each primer in the set has a $T_m$ within the range of from 65 to 74°C, and preferably within the range of from 67 to 74°C. In addition, the primer $T_m$'s are within 5°C of each other, and preferably they differ by no more than 2°C. Further still, the $T_m$'s of the primers in each additional set differ from the $T_m$'s of all other primers in the other sets of primers used in the method by no more than 5°C, and preferably by no more than 2°C. The additional primers also hybridize to nucleic acid sequences in the opposing strands of the particular target nucleic acid, which sequences are spaced apart along the strands by from 90 to 400 nucleotides (more preferably, from 100 to 300 nucleotides apart).

These characteristics and relationships among all of the primers allow for effective and efficient multiplexing using the same PCR processing equipment and conditions.

$T_m$ (melting temperature) is defined herein as the temperature at which one-half of a double stranded DNA molecule is denatured. The determination of $T_m$ can be accomplished using several standard procedures, based on ultraviolet hypochromism, for example, by monitoring the spectrum at 260 nm as described in Biochemistry- The Molecular Basis of Cell Structure and Function, 2nd Edition, Lehninger, Worth Publishers, Inc., 1970, pp. 876-7. The various methods of determining $T_m$ values may produce slightly differing values for the same DNA molecule, but those values should not vary from each other by more than 2 or 3°C.

Preferably, the $T_m$ values described herein for the primers and probes are calculated using the formula (I):

(I)     $T_m (°C) = 67.5 + 0.34(\% G + C) - 395/N$

wherein "G" and "C" represent the number of guanine and cytosine nucleotides, respectively, and "N" represents the total number of nucleotides in the oligonucleotide (that is, primer or probe). $T_m$ values obtained by this calculation correlate very well with the values determined empirically at room temperature using conventional UV hypochromism and a conventional Hewlett-Packard diode array spectrophotometer (scanning rate of +1°C/min.) for a solution of oligonucleotide (primer or probe) in 10 $\mu$molar tris-(hydroxymethyl)aminomethane buffer (pH 8.5) having an ionic strength of at least 60 $\mu$molar provided by one or more inorganic or organic salts, such as magnesium chloride, magnesium sulfate, potassium chloride, sodium chloride, and others readily apparent to one skilled in the art. The amount of oligonucleotide and its complement in the solution used to determine the noted formula was sufficient to provide an optical density of from 0.5 to 1.0 OD units.

Primers useful for the amplification and detection of HIV-I DNA include those having the sequences in the seven primer sets shown below with the $T_m$ in parenthesis:

Primer set 1:

SEQ ID:NO:1      5'-AGTGGGGGGA CATCAAGCAG CCATGCAA- 3'
                      (72.8°C)

SEQ ID:NO:2      5'-TTCCTGCTAT GTCACTTCCC CTTGGTTC-3'
                      (70.4°C),

Primer set 2:

SEQ ID:NO:3      5'-TAGCACCCAC CAGGGCAAAG AGAAGAGT-3'
                      (71.6°C)

SEQ ID:NO:4      5'-AGATGCTGTT GCGCCTCAAT AGCCCTCA-3'
                      (72.1°C),

Primer set 3:

SEQ ID:NO:1      5'-AGTGGGGGGA CATCAAGCAG CCATGCAA-3'
                      (72.8°C)

SEQ ID:NO:5      5'-CTTGGTTCTC TCATCTGGCC TGGTGC-3'
                      (71.6°C),

Primer set 4:

SEQ ID:NO:1      5'-AGTGGGGGGA CATCAAGCAG CCATGCAA-3'
                      (72.8°C)

SEQ ID:NO:13     5'-CCTGCTATGT CACTTCCCCT TGGTTCTCTC-3'
                      (72.5°C),

Primer set 5:

SEQ ID:NO:20     5'-CGTCGTCGTA TAATCCACCT ATCCCAGTAG
                      GAGAAAT-3' (71.3°C),

SEQ ID:NO:21     5'-CGTCGTCGTT TTGGTCCTTG TCTTATGTCC
                      AGAATGC-3'  (73.4°C),

Primer set 6:

SEQ ID:NO:22     5'-ATAATCCACC TATCCCAGTA GGAGAAAT-3'
                      (66.8°C),

SEQ ID:NO:23     5'-TTTGGTCCTT GTCTTATGTC CAGAATGC-3'
                      (68.0°C), and

Primer set 7:

SEQ ID:NO:24     5'-GATGGATGAC AAATAATCCA CCTATCCCAG
                      TAGGAGAAAT-3'  (71.2°C),

SEQ ID:NO:25     5'-CTAAAGGGTT CCTTTGGTCC TTGTCTTATG
                      TCCAGAATGC-3'  (72.9°C).

The primers of sets 1 and 3-7 are complementary to nucleic acid sequences in the "gag" region of HIV-I DNA, and the primers in set 2 are complementary to nucleic acid sequences in the "env" region of

7

HIV-I DNA. Each primer in each set is not limited to use in that set, but can be used with any primer specific to HIV-I DNA that meets the requirements for primers described herein.

Two primer sets useful for the amplification of nucleic acid sequences in opposing strands of HIV-II DNA have the following sequences (and $T_m$'s):

```
          Primer set 8:
   SEQ ID:NO:14    5'-AAGTAGACCA ACAGCACCAC CTAGCGG-3'
                          (71.8°C)
   SEQ ID:NO:15    5'-GCAGCCTTCT GAGAGTGCCT GAAATCCTG-3'
                          (72.6°C), and

          Primer set 9:
   SEQ ID:NO:16    5'-GGGATAGTGC AGCAACAGCA ACAGCTGT-3
                          (71.6°C)
   SEQ ID:NO:17    5'-GTGGCAGACT TGTCTAAACG CACATCCCC-3'
                          (72.6°C).
```

Primers of particular usefulness in the amplification and detection of hCMV DNA include those having the sequences in the three primer sets shown below with the $T_m$ in parenthesis:

```
          Primer set 10:
   SEQ ID NO:46:   5'-GAGGCTATTG TAGCCTACAC TTTGG-3'
                          (68.0°C)
   SEQ ID NO:47:   5'-CAGCACCATC CTCCTCTTCC TCTGG-3'
                          (72.1°C),



          Primer set 11:
   SEQ ID:NO:38    5'-CATTCCCACT GACTTTCTGA CGCACGT-3'
                          (70.5°C)
   SEQ ID:NO:48    5'-TGAGGTCGTG GAACTTGATG GCGT-3'
                          (69.4°C),
       and
          Primer set 12:
   SEQ ID NO:10:   5'-TGCACTGCCA GGTGCTTCGG CTCAT-3'
                          (72.1°C)
   SEQ ID NO:11:   5'-CACCACGCAG CGGCCCTTGA TGTTT-3'
                          (72.1°C).
```

The primers of Set 10 are complementary to nucleic acid sequences in the "major immediate early" region of hCMV DNA, the primers in Set 11 are complementary to nucleic acid sequences in the "major capsid protein" region of hCMV DNA, and the primers in Set 12 are complementary to nucleic acid sequences in the "late antigen" region of hCMV DNA. The primers noted above are not limited in use to the particular set, but can be used with any primer for hCMV DNA which has the properties noted herein.

Matched primers useful for the amplification of human papilloma virus (hPV) DNA include:

Primer set 13:

SEQ ID:NO:26    5'-GAGATGGGAA TCCATATGCT GTATGTGAT-3'
(68°C)

SEQ ID:NO:27    5'-GGACACAGTG GCTTTTGACA GTTAATACA-3'
(68°C),

Primer set 14:

SEQ ID:NO:28    5'-GATGGTCCAG CTGGACAAGC AGAAC-3'
(70.7°C)

SEQ ID:NO:29    5'-CCTAGTGTGC CCATTAACAG GTCTTC-3'
(69.3°C),

Primer set 15:

SEQ ID:NO:30    5'-GACACAGAAA ATGCTAGTGC TTATGCAGC-3'
(69.1°C)

SEQ ID:NO:31    5'-GGTGGACAAT CACCTGGATT TACTGCAAC-3'
(70.3°C),

Primer set 16:

SEQ ID:NO:32    5'-CCTGATCTGT GCACGGAACT GAACACT-3'
(70.5°C)

SEQ ID:NO:33   5'-CCCAGTGTTA GTTAGTTTTT CCAATGTGTC TG-3'
(69°C),

Primer set 17:

SEQ ID:NO:34    5'-TGCCTGCGGT GCCAGAAACC GTTGAAT-3'
(71.8°C)

SEQ ID:NO:35    5'-TGCTCGGTTG CAGCACGAAT GGCACT-3'
(71.9°C),

Primer set 18:

SEQ ID:NO:36    5'-GAGCCGAACC ACAACGTCAC ACAATGTT-3'
(70.4°C)

SEQ ID:NO:37    5'-GGACACACAA AGGACAGGGT GTTCAGAAA-3'
(70.3°C), and

Primer set 19:

SEQ ID:NO:37    5'-GGACACACAA AGGACAGGGT GTTCAGAAA-3'
(70.3°C)

SEQ ID:NO:39    5'-GCGACTCAGA GGAAGAAAAC GATG-3'
(68°C).

Matched primers useful for the amplification o*f Mycobacterium tuberculosis* (Mtb) DNA include:

9

Primer set 20:
SEQ ID:NO:40    5'-GAGATCGAGC TGGAGGATCC GTACG-3'
(72.1°C)

SEQ ID:NO:41    5'-AGCTGCAGCC CAAAGGTGTT GGACT-3'
(70.7°C), and

Primer set 21:
SEQ ID:NO:42    5'-TCAGCCGCGT CCACGCCGCG A-3'
(75°C)

SEQ ID:NO:43    5'-CCTGCGAGCG TAGGCGTCGG-3'
(73.3°C).

SEQ ID:NO:42 is slightly outside the claimed range of matched primers, but PCR is still possible using it, although not as efficiently for "multiplexing".

A matched primer set useful for the amplification of *Mycobacterium avium* (Mav) DNA, is as follows:

Primer set 22:
SEQ ID:NO:44    5'-GAGATCGCCA CCTTCGGCAA-3'
(68.2°C)

SEQ ID:NO:45    5'-GAGCAGTTCG GTGGCGTTCA-3'
(68.2°C).

A matched primer set useful for the amplification of the thymidine kinase gene of Herpes simplex virus 1 (HSV-1) DNA is as follows:

Primer set 23:
SEQ ID:NO:63    5'-CCGGGAGATG GGGGAGGCTA ACTGA-3'
(73.5°C)

SEQ ID:NO:64    5'-GGGGTGGGGA AAAGGAAGAA ACGCG-3'
(72.1°C).

Primers useful for the amplification and detection of additional target nucleic acids would be readily determinable by a skilled worker in the art by consultation with the considerable literature in this field to determine appropriate nucleic acid sequences of target nucleic acids. Those sequences can then be used as patterns for preparing primers using known technology. The primers can be readily screened by determining if they have the requisite $T_m$ (using appropriate methods defined above) and other requirements as defined above.

As used herein, a "probe" is an oligonucleotide which is substantially complementary to a nucleic acid sequence of the target nucleic acid (for example, HIV-I DNA or any additional target nucleic acid) and which is used for detection or capture of the amplified target nucleic acid. The probes generally have from 10 to 40 nucleotides, and a $T_m$ greater than 50°C. Moreover, the probes are hybridizable with a nucleic acid sequence of the particular target nucleic acid at a temperature in the range of from 40 to 55°C (preferably in the range of from 45 to 53°C). In the use of a multiplicity of probes for simultaneously capturing a multiplicity of amplified target nucleic acids in the practice of this invention, all of the capture probes have $T_m$'s which differ by no more than 15°C. Preferably, the capture probe $T_m$'s used simultaneously differ by no more than 5°C.

Representative capture probes for HIV-I DNA include the following oligonucleotides, with the $T_m$'s in parenthesis:

```
SEQ ID:NO:6     5'-GAGACCATCA ATGAGGAAGC TGCAGAAT-3'
                                           (69.2°C), and

SEQ ID:NO:7     5'-GTGCAGCAGC AGAACAATTT GCTGAGGG-3'
                                           (71.6°C).
```

The first listed probe is complementary to a nucleic acid sequence in the the "gag" region of HIV-I DNA, and the second listed probe is complementary to a nucleic acid sequence in the "env" region of HIV-I DNA.

Representative capture probes useful in the detection of an amplified nucleic acid sequence of HIV-II DNA include the following (with $T_m$):

```
SEQ ID:NO:18    5'-GAGGAAAAGA AGTTCGGGGC AGAAGT-3'
                (69.3°C), and

SEQ ID:NO:19    5'-CAACAAGAAA TGTTGCGACT GACCGTCT-3'
                (69.2°C).
```

Representative useful capture probes for hCMV DNA include the following oligonucleotides, with the $T_m$ in parenthesis:

```
SEQ ID:NO:8     5'-GGTGTCACCC CCAGAGTCCC CTGTACCCGC-3'
                                           (78.1°C),

SEQ ID:NO:49    5'-GACACAGTGT CCTCCCGCTC CTCCTGAGCA-3'
                                           (75.9°C),

SEQ ID:NO:50    5'-GTGGAAGGCG GCTCGCTGGA AGCCGGTCGT-3'
                                           (78.1°C),

SEQ ID:NO:12    5'-GAACCGAGGG CCGGCTCACC TCTATGTTGG-3'
                                           (75.9°C), and

SEQ ID:NO:62    5'-GGTCATCGCC GTAGTAGATG CGTAAGGCCT-3'
                                           (73.6°C).
```

The first two listed probes are complementary to nucleic acid sequences in the "major immediate early" region of hCMV DNA, the next two listed probes are complementary to nucleic acid sequences in the "late antigen" region of hCMV DNA, and the last probe is complementary to a nucleic acid sequence in the "major capsid protein" region of hCMV DNA.

Representative probes useful for the detection of human papilloma viral (hPV) DNA include:

```
SEQ ID:NO:51 5'-GGAACAACAT TAGAACAGCA ATACAACAAA CCG-3'
                                           (68.9°C),
SEQ ID:NO:52  5'-AATATTGTAA CCTTTTGTTG CAAGTGTGAC TC-3'
                                           (66.8°C),
SEQ ID:NO:53   5'-CCTATAGGTG GTTTGCAACC AATTAAACAC-3'
                                           (67.9°C),
SEQ ID:NO:54   5'-GAGGTATTTG AATTTGCATT TAAAGATTTA
                      TTTGT-3'        (63.8°C),


  SEQ ID:NO:55   5'-GCAAGACAGT ATTGGAACTT ACAGAGG-3'
                                           (68°C), and
  SEQ ID:NO:56   5'-GTGTTGTAAG TGTGAAGCCA GATTTGA-3'
                                           (66.7°C).
```

Capture probes useful for the detection of *Mycobacterium tuberculosis* (Mtb) DNA include:

```
  SEQ ID:NO:57   5'-GAGCAGATTG CGGCCACCGC AGCGATTTCG-3'
                                           (75.9°C), and
  SEQ ID:NO:58   5'-CTCGTCCAGC GCCGCTTCGG-3' (73.3°C).
```

Useful capture probes for the detection of *Mycobacterium avium* (Mav) DNA include:

```
  SEQ ID:NO:59   5'-TGGATCTCGT TGTTCGGGTC-3' (66.5°C),
  and
  SEQ ID:NO:60   5'-GAGCAGATCG CTGCCACCGC CGGTATCTCC-3'
                                           (78.1°C).
```

A useful capture probe for the detection of *Mycobacterium fortuitum* DNA is:

```
  SEQ ID:NO:61   5'-GAGCAGATCG CTGCCACCGC CGGTATCTCC-3'
                                           (77°C).
```

A useful capture probe for the detection of the thymidine kinase gene of herpes simple virus 1 (HSV-1) DNA is as follows:

```
  SEQ ID:NO:65 5'-AAAGACAGAA TAAAACGCAC GGGTGTTGGG TCG-3'
                                           (70.2°C).
```

Probes useful for the detection or capture of additional target nucleic acids would be readily apparent to one skilled in the art if the targeted nucleic acid sequences are known. Thus, the practice of this invention is adequately enabled by knowing those sequences and following the representative teaching herein regarding primers and probes actually shown. Presently, unknown target nucleic acids will also be similarly amplified and detected because this technology could predictably be used in a similar fashion. Potential probes can be screened to see if they have the requisite $T_m$ as defined above. Such probes can be prepared using the same procedures and starting reagents described for primers above.

Additional PCR reagents necessary for PCR include a thermostable DNA polymerase, a DNA polymerase cofactor and appropriate dNTP's. These reagents can be provided individually, as part of a test kit, in reagent chambers of a test device, or in the composition of this invention.

A thermostable DNA polymerase is an enzyme which will add deoxynucleoside monophosphate molecules to the 3' hydroxy end of the primer in a complex of primer and template. Synthesis of extension products proceeds in the 5' to 3' direction of the newly synthesized strand until synthesis is terminated.

The DNA polymerase is "thermostable" meaning that it is stable to heat and preferentially active at higher temperatures, especially the high temperatures used for priming and extension of DNA strands.

A number of thermostable DNA polymerases have been reported in the art, including those mentioned in detail in US-A-4,965,188 and US-A-4,889,818. Particularly useful polymerases are those obtained from various Thermus bacterial species, such as *Thermus aquaticus, Thermus thermophilus, Thermus filiformis* or *Thermus flavus.* Other useful thermostable polymerases are obtained from a variety of other microbial sources including *Thermococcus literalis, Pyrococcus furiosus,* Thermotoga sp. and those described in WO-A-89/06691.

A DNA polymerase cofactor refers to a nonprotein compound on which the enzyme depends for activity. A number of such materials are known cofactors including manganese or magnesium in such a form that divalent cations are released into an aqueous solution. Useful cofactors include manganese and magnesium salts, such as chlorides, sulfates, acetates and fatty acid salts (for example, butyric, caproic, caprylic, capric and lauric acid salts).

Also needed for PCR are two or more deoxyribonucleoside-5'-triphosphates (dNTP's), such as dATP, dCTP, dGTP, dTTP or dUTP. Analogues such as dITP and 7-deaza-dGTP are also useful. The preferred materials, dATP, dCTP, dGTP and dTTP, are used collectively in the assays.

The PCR reagents described herein are provided and used in PCR in any concentration suitable for a given process. Preferably, from 0.1 to 50 units of thermostable DNA polymerase per 100 $\mu$l of reaction mixture are used for PCR, depending upon the particular activity of a given enzyme. A "unit" is defined herein as the amount of enzyme activity required to incorporate 10 nmoles of total nucleotides (dNTP's) into an extending nucleic acid chain in 30 minutes at 74 °C. More preferably, from 10 to 40 units of DNA polymerase/100 $\mu$l of solution are used. The amount of primer is at least 0.075 $\mu$molar with from 0.1 to 2 $\mu$molar being preferred. Preferably, each primer is present in the same amount. The cofactor is generally present in an amount of from 2 to 15 $\mu$molar. Each dNTP is present at from 0.25 to 3.5 $\mu$molar (1 to 14 $\mu$molar for total of four common dNTP's).

The aqueous composition of this invention is buffered to a pH of from 7 to 9 (preferably from 8 to 8.5) using one or more suitable buffers.

A particularly useful composition of this invention is a buffered mixture of the primers noted herein, a magnesium cofactor as noted above, each of dATP, dCTP, dGTP and dTTP as noted above, gelatin or a similar hydrophilic colloidal material (in an amount of at least 5%, by weight), and an alkali metal salt (such as sodium chloride or potassium chloride) present in an amount of from 10 to 100 $\mu$molar.

In one embodiment of this invention, a method for preparing a reaction mixture for polymerase chain reaction of two or more target DNA's comprises:

A) choosing a set of primers for each distinct target DNA, the primers in each set chosen to be specific to and hybridizable with nucleic acid sequences which are in opposing strands of a distinct target DNA and which are separated from each other along the opposing strands of the distinct target DNA by from 90 to 400 nucleotides

each of the primers in each primer set having a $T_m$ within the range of from 65 to 74 °C, all of the primer $T_m$'s being within 5 °C of each other, and the primers in each set having nucleotide lengths which differ from each other by no more than 5 nucleotides,

the $T_m$'s being calculated using the formula:

$$T_m \,(°C) = 67.5 + 0.34(\%G + C) - 395/N$$

wherein G and C represent the number of guanine and cytosine nucleotides, respectively, and N represents the total number of nucleotides, and

B) mixing the sets of primers chosen in step A) with:

a thermostable DNA polymerase in an amount of from 0.1 to 50 units/100 $\mu$l,

a DNA polymerase cofactor in an amount of from 2 to 15 $\mu$molar, and

each of dATP, dCTP, dGTP and dTTP present in an amount of from 0.25 to 3.5 $\mu$molar,

13

wherein each of the primers is present in the mixture at a concentration of at least 0.075 μmolar.

A target nucleic acid can be obtained from any of a variety of sources as noted above, such as a whole blood sample. Generally, it is extracted in some manner to make it available for contact with the primers and other PCR reagents. This usually means removing unwanted proteins and cellular matter from the specimen in a suitable manner.

Since the nucleic acid to be amplified and detected is usually in double stranded form, the two strands must be separated (that is, denatured) before priming can take place. This can occur during the extraction process, or be a separate step afterwards. Denaturation is accomplished using a heat treatment alone or in combination with any suitable other physical, chemical or enzymatic means as described in the art. Initial denaturation is generally carried out by heating the specimen suspected of containing the targeted nucleic acid at a first temperature of from 85 to 100°C for a suitable time, for example from 1 second to 3 minutes.

The denatured strands are then cooled to a second temperature which is generally in the range of from 55 to 70°C for priming the strands. The time needed for cooling the denatured strands will vary depending upon the type of apparatus used for the PCR process.

Once the denatured strands are cooled to the second temperature, the reaction mixture containing PCR reagents is incubated at a suitable temperature to effect formation of primer extension products. Generally, this temperature is at least 50°C, and preferably in the range of from 62 to 75°C. The time for incubation can vary widely depending upon the incubation temperature and the length of extension products desired, but in preferred embodiments, it is from 1 to 120 seconds. Each cycle of PCR can be carried out using either two or three different temperatures, one for denaturation, and a second and/or third temperature for priming and/or primer extension product formation. That is, some PCR processes utilize a second temperature for priming and a third temperature for primer extension. Preferably, in the practice of this invention, the same temperature is used for both priming and primer extension.

If the hybridized primer extension products are then denatured, PCR can be carried out further in as many cycles of priming, extension and denaturation as desired. Generally, at least 20 cycles will be carried out, with from 20 to 50 cycles being preferred.

One such instrument useful for PCR is described in US-A-4,965,188 and EP-A-0 236 069. Generally, this instrument includes a heat conducting container for holding a number of reaction tubes containing reaction mixture, a means for heating, cooling and temperature maintenance, and a computing means to generate signals to control the amplification process, changes in temperature and timing.

A preferred instrument for processing amplification reactions in a disposable chemical test pack is described in some detail in US-A-5,089,233 (Devaney et al). In general, this instrument comprises a surface for supporting one or more chemical test packs, pressure applicators supported above the surface for acting on the reaction pack to transfer fluids between adjacent chambers in the test pack, and means for operating the pressure applicators through a range of movements extending across the test pack.

It is also useful for the method of this invention to be carried out in a suitable container. The most crude container would be a test tube, cuvette, flask or beaker, but more sophisticated containers have been fashioned in order to facilitate automated procedures for performing the method (see for example, WO-A-91/12342). For example, cuvette and chemical test packs (also known as pouches), constructed to provide certain temperature characteristics during the practice of the method, are described in US-A-4,902,624, US-A-5,173,260 and US-A-5,229,297. Such test packs have a multiplicity of reagent chambers having various reagents, buffers and other materials which are useful at various stages in the amplification or detection method. The aqueous composition of this invention can be incorporated into a reaction chamber for use in PCR. The packs can be appropriately and rapidly heated and cooled in cycles to promote the various steps of the amplification method of this invention.

Detection of the amplified target DNA's can be accomplished in a number of known ways, such as those described in US-A-4,965,188. For example, it can be detected using Southern blotting or dot blot techniques. Alternatively, amplification can be carried out using primers that are appropriately labeled (such as with a radioisotope), and the amplified primer extension products are detected using procedures and equipment for detection of radioisotopic emissions.

In one embodiment, the amplified target nucleic acid is detected using an oligonucleotide probe which is labeled for detection and can be directly or indirectly hybridized with one of the primer extension products. Procedures for attaching labels and preparing probes are well known in the art, for example, as described by Agrawal et al, *Nucleic Acid Res.*, 14, pp. 6227-45 (1986), US-A-4,914,210, US-A-4,962, 029, and the references noted therein. Useful labels include radioisotopes, electron-dense reagents, chromogens, fluorogens, phosphorescent moieties, ferritin and other magnetic particles (see US-A-4,795,698 and US-A-4,920,061, chemiluminescent moieties and enzymes. Useful enzymes include, glucose oxidase, peroxidases, uricase, alkaline phosphatase and others known in the art and can be attached to oligonucleotides using known procedures. Substrate reagents which provide a chemiluminescent or colorimetric signal in the presence of a particular enzyme label would be readily apparent to one skilled in the art.

Where the label is a preferred enzyme such as a peroxidase, at some point in the assay, hydrogen peroxide and a suitable dye-forming composition are added to provide a detectable dye (that is, a colorimetric signal). For example, useful dye-providing reagents include tetramethylbenzidine and derivatives thereof, and leuco dyes, such as triarylimidazole leuco dyes (as described in US-A-4,089,747), or other compounds which react to provide a dye in the presence of peroxidase and an oxidant such as hydrogen peroxide. Particularly useful dye-providing compositions are described in US-A-5,024,935. Chemiluminescent signals can be generated using acridinium salts or luminol and similar compounds in combination with enhancers in the presence of peroxidase.

In a preferred embodiment, one or both of the primers in each primer set used to detect a target nucleic acid is labeled with a specific binding moiety. The specific binding moiety can be the same or different for each set of primers. Such labels include any molecule for which there is a receptor molecule that reacts specifically with the specific binding moiety. Examples of specific binding pairs (one of which can be the label) include avidin/biotin, streptavidin/biotin, sugar/lectin, antibody/hapten and antibody/antigen. The receptor is then conjugated with a detectable label moiety, such as an enzyme using known technology.

Most preferably, one or both primers of each primer set are labeled with biotin (or a equivalent derivative thereof), and the amplified target nucleic acid is detected using a conjugate of avidin (or streptavidin) with an enzyme.

In order for the amplified target nucleic acid to be detected, it is often useful for it to be separated from the other materials in the reaction medium. This is done by any of a number of ways, including using a capture reagent having a capture probe which is covalently attached to a water-insoluble support. The capture probe hybridizes with the amplified target nucleic acid and the captured material can then be separated from unhybridized materials in a suitable manner, such as by filtration, centrifugation, washing or other suitable separation techniques.

Capture probes can be attached to water-insoluble supports using known attachment techniques. One such technique is described in EP-A-0 439 222. Other techniques are described for example in US-A-4,713,326, US-A-4,914,210 and EP-B-0 070 687. Useful separation means are microporous filtration membranes such as the polyamide membranes marketed by Pall Corp. which can be used to separate captured target nucleic acids from unhybridized materials.

Any useful solid support can be used for separation of water-insoluble product for detection, including a microtiter plate, test tube, beaker, beads, film, membrane filters, filter papers, gels, magnetic particles or glass wool. It can be made of a number of materials including glass, ceramics, metals, naturally occurring or synthetic polymers, cellulosic materials, filter materials and others readily apparent to one of ordinary skill in the art. Particularly useful solid support materials are polymeric or magnetic particles generally having an average particle size of from 0.001 to 10 $\mu$meters. Further details about such preferred polymeric particles are provided in US-A-4,997,772, US-A-5,147,777, US-A-5,155,166.

The detection can also be carried out by immobilizing a capture probe or capture reagent on a flat substrate, such as the microporous filtration membranes described above, or on thin polymeric films, uncoated papers or polymer coated papers, a number of which are known in the art. Other details about such materials are provided in EP-A-0 408 738.

Particularly useful arrangements of a capture reagent are described, for example, in WO 92/16659 and US-A-5,173,260. The capture probes are covalently attached (either directly or through chemical linking groups) to the same type of polymeric particles, and the resulting capture reagents are immobilized on a heat or ultrasonic sealable support (for example, a sheet, membrane, fibrous mat, film). One particularly useful sealable support is a laminate of polyethylene and a polyester such as polyethylene terephthalate. The capture reagents can be disposed in distinct regions on the water-insoluble support which is part of a suitable test device (as described above). Such test devices can also be defined as diagnostic elements. For example, the support can have disposed thereon a plurality of stripes or spots of various capture reagents. The multiplicity of capture probes arranged in defined regions on such supports all have the $T_m$ values as described above, that is the $T_m$ values differ by no more than 15 °C (preferably by no more than 5 °C).

Thus, according to one embodiment of this invention, a diagnostic element comprises a water-insoluble, heat or ultrasonic sealable support, having disposed thereon in distinct regions thereof, a plurality (two or more) of capture reagents,

each of the capture reagents having a capture probe specific for and hybridizable with a distinct (that is, unique to that capture probe) target DNA associated with an infectious agent at a temperature of from 40 to 55°C, each of the capture probes having from 10 to 40 nucleotides and a $T_m$ greater than 50°C, and the $T_m$'s of all capture probes differing by no more than 15°C.

The present invention includes diagnostic test kits which can include the composition of this invention, an additional PCR reagent and other materials, equipment and instructions needed to carry out the method of the invention. The kits can include one or more detection or capture probes, multiple primer sets and test devices for the assays. In some embodiments, the kit components are separately packaged for use in a suitable container or test device. In other embodiments, the kit contains a test device having within separate compartments, some or all of the reagents and compositions needed for the assay. In such embodiments, the separate packaging of the kit components can be within a single test device.

Materials and Methods for Examples:

Recombinant DNA polymerase from *Thermus aquaticus* was prepared using known procedures, such as that described in EP-A-0 482 714 and had an activity of 250,000 units/mg of protein.

The primers and probes were prepared using known starting materials and procedures using an Applied Biosystems Model 380B, three column DNA synthesizer using standard phosphoramidite chemistry and the ABI 1 $\mu$molar scale, fast cycle protocol. Nucleoside-3'-phosphoramidites and nucleoside derivatized controlled pore glass supports were obtained from Applied Biosystems. The primers had the sequences identified above. They were functionalized at the 5' end with two tetraethylene glycol spacers followed by a single commercially available DuPont biotin phosphoramidite. The probes were functionalized at the 3' end with two tetraethylene glycol spacers followed by a single aminodiol linking group according to US-A-4,914,210. All purifications were carried out using a nucleic acid purification column, followed by reversed phase HPLC techniques.

The monoclonal antibody specific to the noted DNA polymerase was prepared as described in EPC Application 93202801.2. Generally, it was prepared from the immune cells of DNA polymerase immunized mice using conventional procedures, such as those described by Milstein et al, *Nature* 256, pp. 495-497, 1975 and hybridoma cell lines (either HB 11126 or 11127 from ATCC), whereby antibody secreting cells of the host animal were isolated from lymphoid tissue (such as the spleen) and fused with SP2/0-Ag14 murine myeloma cells in the presence of polyethylene glycol, diluted into selective media and plated in multiwell tissue culture dishes. About 7-14 days later, the hybridoma cells containing the antibodies were harvested, and purified using conventional techniques.

An avidin-peroxidase conjugate solution comprised a commercially available (Zymed Laboratories, Inc.) conjugate of avidin and horseradish peroxidase (126 $\mu$l/l), casein (0.5%) and merthiolate (0.5%).

A wash solution (pH 7.4) contained sodium phosphate, monobasic 1-hydrate (25 $\mu$molar), sodium chloride (373 $\mu$molar), (ethylenedinitrilo)tetracetic acid disodium salt (2.5 $\mu$molar), ethylmercurithiosalicylic acid sodium salt (25 $\mu$molar), and decyl sodium sulfate (38 $\mu$molar).

The dye-providing composition (pH 6.8) contained 4,5-bis(4-dimethylaminophenyl)-2-(4-hydroxy-3-methoxyphenyl)imidazole (250 $\mu$molar), poly(vinyl pyrrolidone) (112 $\mu$molar), agarose (0.5%), diethylenetriaminepentaacetic acid (100 $\mu$molar), 4'-hydroxyacetanilide (5 $\mu$molar) and sodium phosphate, monobasic, 1-hydrate (10 $\mu$molar).

HIV-I DNA was extracted from the HUT/AAV 78 cell line using conventional procedures, and following cell lysis and protein digestion, was purified by phenol/chloroform extraction: tris-saturated phenol (750 $\mu$l) was added to the cell suspension, and phenol/lysate solutions were mixed and separated by centrifugation. The aqueous phase was then transferred into a fresh 2 ml tube. This procedure was repeated using chloroform isoamyl alcohol. The aqueous layer was brought to 0.3 molar sodium acetate. Nucleic acids were precipitated by adding 95% cold ethanol and storing at -70°C for 1 hour. The concentration of HIV-I DNA was then determined at $A_{260}$ and serial dilutions of varying copy number were made in TE buffer [tris-(hydroxymethyl)aminomethane (1 $\mu$molar) and (ethylenedinitrilo)tetraacetic acid (0.1 $\mu$molar)] for experimental use. A sample (10 $\mu$l) of the diluted solutions was added to each PCR reaction mixture (300 $\mu$l).

Pure hCMV DNA was obtained by purifying commercially available crude hCMV DNA (Advanced Biotech's strain AD169) using a conventional sucrose gradient and phenol/chloroform extraction procedures. The concentration of hCMV DNA was then determined at $A_{260}$ and target dilutions of varying calculations of copy number were made for experimental use in TE buffer [tris(hydroxymethyl)aminomethane (1 $\mu$molar), ethylenediaminetetraacetic acid (0.1 $\mu$molar)]. A sample (10 $\mu$l) of the diluted solutions was added to 300 $\mu$l of PCR reaction mixture.

Two "nonsense" probes were used as control reagents for the assays to amplify and detect HIV-I DNA and had the sequences:

```
SEQ ID:NO:8
           5'-GGTGTCACCC CCAGAGTCCC CTGTACCCGC-3'
    SEQ ID:NO:9
      5'-ATCCTGGGAT TAAATAAAAT AGTAAGAATG TATAGCCCTA C-3'
```

The same "nonsense" probes were used as controls for the hCMV DNA assays also.

Capture reagents were prepared by attaching the capture probes identified above to particles of poly-[styrene-co-3-(p-vinylbenzylthio)propionic acid] (95:5 molar ratio, 1 μm average diameter) in the following manner. A suspension of the particles in water was washed twice with 2-(N-morpholino)ethanesulfonic acid buffer (0.1 molar, pH 6), and suspended to approximately 10% solids. A sample (3.3 ml) of the washed particles, diluted to 3.33% solids in the buffer (0.1 molar), was mixed with 1-(3-dimethylaminopropyl)-3-ethylcarbodiimide hydrochloride (2.1 ml of 84 mg/ml water) and the appropriate probe (983 μl of 44.44 OD/ml nanopure water). The resulting suspension was heated at 50°C in a water bath for about two hours with intermittent mixing and centrifuged. The particles were washed three times with tris (hydroxy-methyl) aminomethane buffer (0.01 molar, pH 8) containing (ethylenedinitrilo)tetraacetic acid disodium salt (0.0001 molar) and resuspended therein to 4% solids.

Capture probes used for the detection of amplified HIV-I DNA were SEQ ID:NO:6 and SEQ ID:NO:7, with the first one being for the "gag" region of HIV-I DNA and the second one for the "env" region of HIV-I DNA.

Control capture reagents were similarly prepared using the "nonsense" probes identified above.

All of the capture reagents were mounted on a heat sealable polyethylene/polyester laminate (treated by corona discharge) in test devices prepared as described in WO-A-92/16659 (noted above) so that the assay fluids and reagents contacted all of the capture reagents at about the same time. PCR was carried out using an automated Kodak PCR processor which is described in detail in US-A-5,089,233.

Primers (and $T_m$) used for the amplification and detection of hCMV DNA were as follows:

```
      SEQ ID:NO:10  5'-TGCACTGCCA GGTGCTTCGG CTCAT-3'
      (72.1°C), and
      SEQ ID:NO:11  5'-CACCACGCAG CGGCCCTTGA TGTTT-3'
      (72.1°C).
```

A capture reagent for hCMV DNA was prepared as described above using the following capture probe ($T_m$):

```
    SEQ ID:NO:12  5'-GAACCGAGGG CCGGCTCACC TCTATGTTGG-3'
                   (75.8°C).
```

In the following examples, all percentages are by weight unless otherwise indicated.

Example 1 Buffered Composition Containing HIV-I DNA Primers

One composition of this invention was prepared by mixing primers with additional PCR reagents. This composition contained tris(hydroxymethyl)aminomethane hydrochloride buffer (10 μmolar, pH 8), tris-(hydroxymethyl)aminomethane buffer (6.86 μmolar), potassium chloride (50 μmolar), ethylenediaminetetraacetic acid (686 μmolar), magnesium chloride (10 μmolar), gelatin (100 μg/ml), dATP, dCTP, dGTP and dTTP (1.5 μmolar of each), glycerol (9.5%), primers (0.4 μmolar of each), DNA polymerase identified above (48 units/300 μl), and a monoclonal antibody specific to DNA polymerase identified above (50:1 molar ratio to DNA polymerase). The primers included were those identified as SEQ ID:NO:1 and SEQ ID:NO:5 which are specific to nucleic acid sequences in the "gag" region of HIV-I DNA, and SEQ ID:NO:3 and SEQ ID:NO:4 which are specific to nucleic acid sequences in the "env" region of HIV-I DNA. The composition also contained phenol/chloroform purified CEM cells (normal uninfected lymphocytes, at either 2.75 or 6 $\mu$g/300 $\mu$l) to simulate a human blood sample.

Example 2 Simultaneous Amplification and Detection of HIV-I DNA and hCMV DNA

This example demonstrates the practice of the present invention using the composition described in Example 1 to simultaneously detect HIV-I DNA along with hCMV DNA, except that the composition further contained 0.4 $\mu$molar of each of the primers identified above as SEQ ID:NO:10 and SEQ ID:NO:11.

Twenty-four assays were carried out to detect the following various concentrations of the target nucleic acids in the test samples having two different amounts of CEM cells:

Sample a) 20,000 copies of hCMV DNA and 20,000 copies of HIV-I DNA,

Sample b) 500 copies of hCMV DNA and 500 copies of HIV-I DNA,

Sample c) 100 copies of hCMV DNA and 100 copies of HIV-I DNA,

Sample d) 100 copies of hCMV DNA and 20,000 copies of HIV-I DNA,

Sample e) 20,000 copies of hCMV DNA and 100 copies of HIV-I DNA, and

Sample f) 100 copies of hCMV DNA and 500 copies of HIV-I DNA.

In these assays, a nucleic acid sequence in the "late antigen" region of hCMV DNA was detected, and nucleic acid sequences in the "gag" and "env" regions of HIV-I DNA were detected. Two replicates were carried out for each assay.

The amplification and detection procedure for the assays were as follows:

Amplification:

Denature by heating at 95°C for 60 seconds,

40 cycles of priming and extending at 68°C for 30 seconds, and heating at 94°C for 15 seconds.

Detection:

Denature the amplified strands at 97°C for 120 seconds,

Capture the amplified products with the capture reagents at 50°C for 5 minutes,

Contact and incubate the captured products with the avidin-peroxidase conjugate solution at 40°C for 1 minute,

Wash the captured products using the wash solution at 40°C for 1 minute,

Add the dye-providing composition and incubate at 40°C for 2 minutes, and

Read the dye signal.

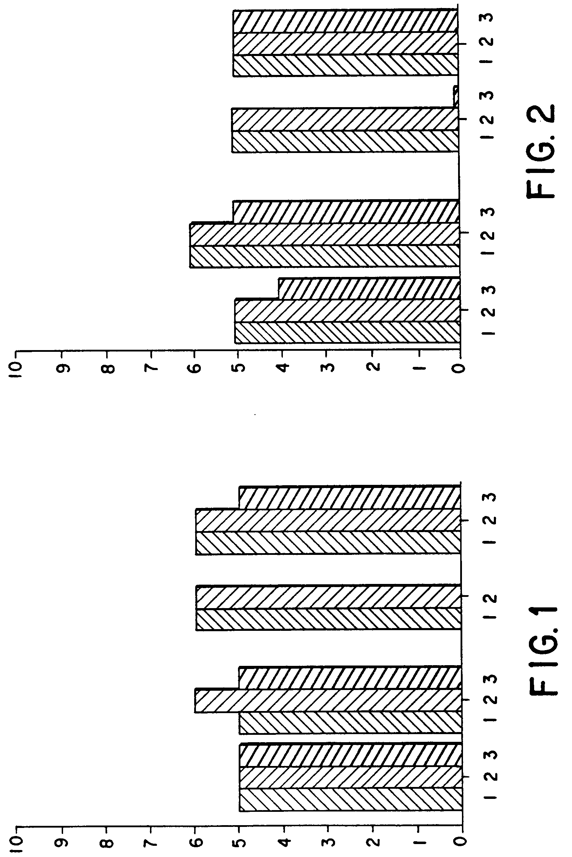

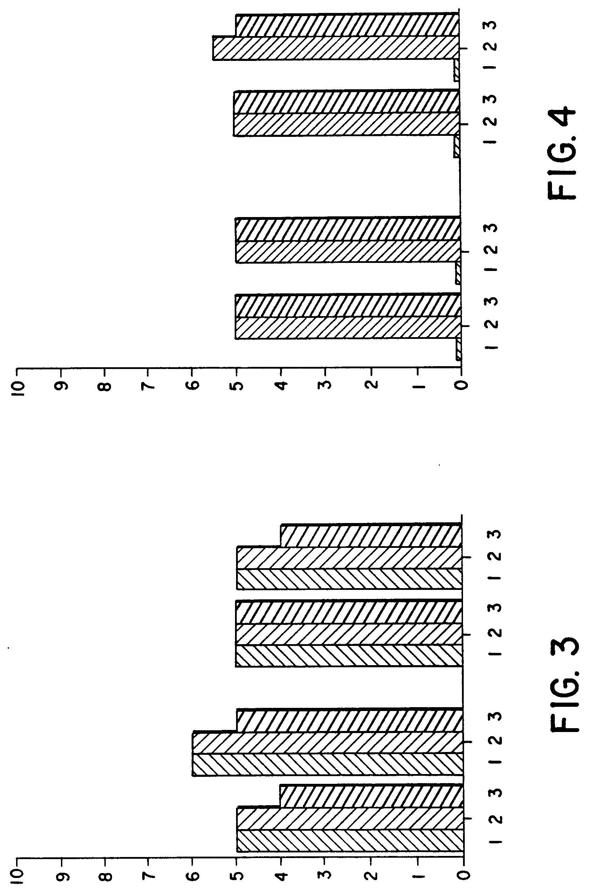

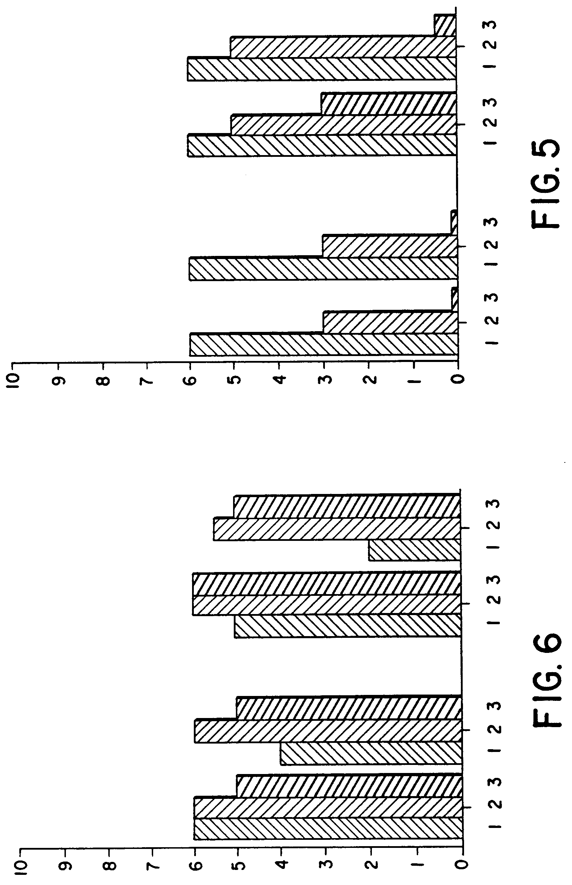

The results of the assays (two replicates of each assay) of Samples a)-f), are shown in the bar graphs of FIGS. 1-6, respectively, where the dye signal is shown in the y-axis (where "0" represents no dye signal, and "10" represents highest dye density). In each figure, the first set of bar graphs are assays whereby 2.75 $\mu$g CEM cells were present, and the second set of bar graphs are assays whereby 6 $\mu$g CEM cells were present. Also, in all figures, the first bar (identified as "1") in each set of bars represents the signal from hCMV DNA ("late antigen" region), the second bar (identified as "2") represents the signal from HIV-I DNA ("gag" region), and the third bar (identified as "3") represents the signal from HIV-I DNA ("env" region). The dye signals for both Control capture reagents were essentially zero, so they are not illustrated on the bar graphs.

Example 3 Amplification and Detection of HIV-I DNA Alone

This example was carried out similarly to Example 2 for the amplification and detection of two nucleic acid sequences of HIV-I DNA ("gag" and "env" regions) only in Samples a)-f) using the composition of Example 1 (6 $\mu$g CEM cells only).

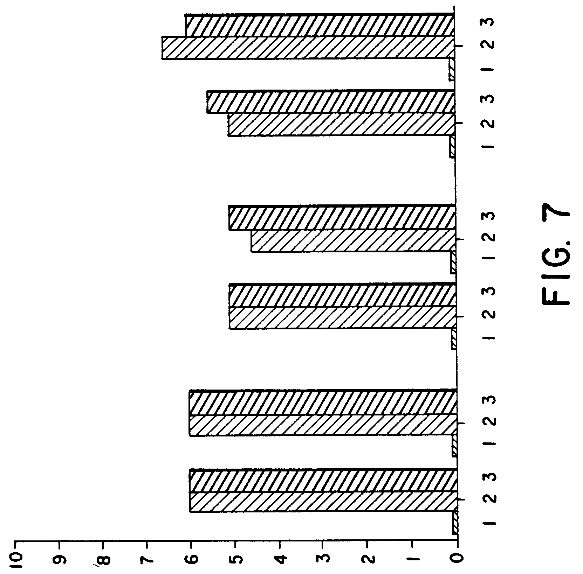

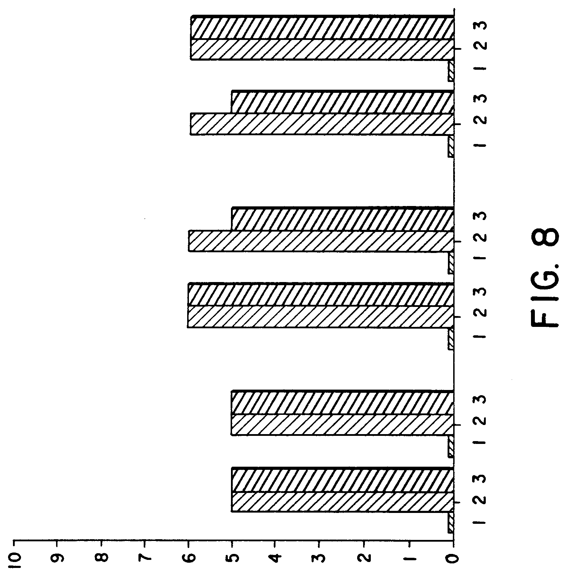

FIG. 7 shows the dye signal results of the PCR process for the two replicates of each of Samples a)-c), and FIG. 8 shows the dye signal results of the PCR process for the replicates of each of Samples d)-f). Clear signals were observed for the presence of HIV-I DNA (bars identified as "2" and "3"). Small background signals were also observed (bar identified as "1" in each set of bar graphs).

Example 4 Buffered Composition Containing hCMV DNA Primers

Another composition of this invention was prepared by mixing primers with additional PCR reagents. This composition contained tris(hydroxymethyl)aminomethane hydrochloride buffer (10 $\mu$molar, pH 8), potassium chloride (50 $\mu$molar), magnesium chloride (10 $\mu$molar), gelatin (100 $\mu$g/ml), dATP, dCTP, dGTP and dTTP (1.5 $\mu$molar of each), glycerol (7.5%), primers (0.4 $\mu$molar of each), DNA polymerase identified above (48 units/300 $\mu$l), and a monoclonal antibody specific to DNA polymerase identified above (50:1 molar ratio to DNA polymerase). The primers included were those identified as SEQ ID:NO:10 and 11 which are specific to nucleic acid sequences of hCMV DNA. The composition also contained phenol/chloroform purified CEM cells (normal uninfected lymphocytes, at either 2.75 or 6 $\mu$g/300 $\mu$l) to simulate a human blood sample.

Example 5 Amplification and Detection of hCMV DNA Alone

This example was carried out similarly to Example 2 for the amplification and detection of hCMV DNA ("late antigen" region only) in Samples a)-f) using the composition of Example 4 (2.75 $\mu$g CEM cells only).

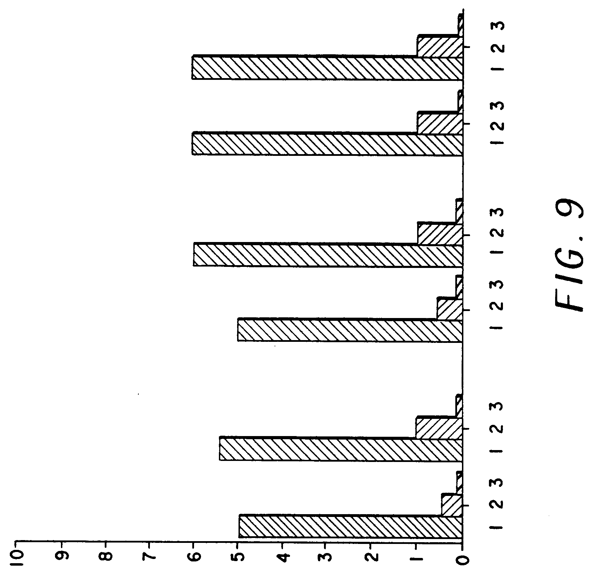

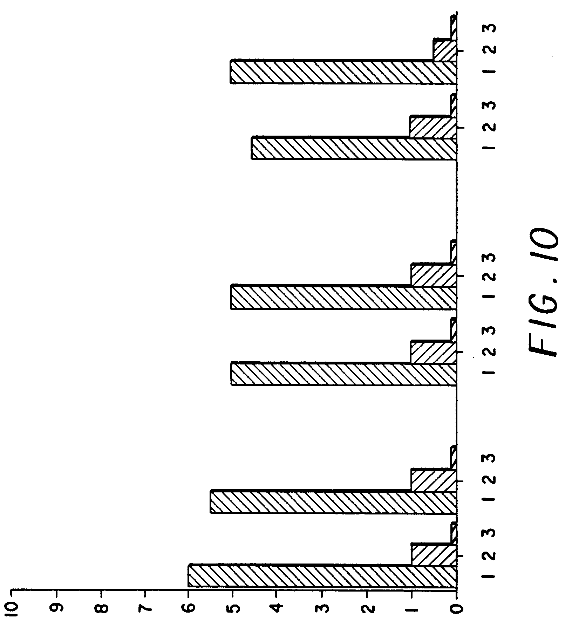

FIG. 9 shows the dye signal results of the PCR process for the two replicates of each of Samples a)-c), and FIG. 10 shows the dye signal results of the PCR process for the replicates of each of Samples d)-f). Clear signals were observed for the presence of hCMV DNA (bar graphs labeled "1"). Small background signals were also observed (labeled as "2" and "3", respectively in each set of bar graphs) from the presence of HIV-I DNA ("gag" and "env" regions).

SEQUENCE LISTING

(1) GENERAL INFORMATION

    (i) APPLICANT:

        (A) NAME:     Eastman Kodak Company

        (B) STREET:   343 State Street

        (C) CITY:     Rochester

        (D) STATE:    New York

        (E) COUNTRY:  USA

        (F) POSTAL CODE: 14650-2201

    (ii) TITLE OF THE INVENTION: DIAGNOSTIC COMPOSITIONS, ELEMENTS, METHODS AND TEST KITS FOR AMPLIFICATION AND DETECTION OF TWO OR MORE TARGET DNA'S USING PRIMERS HAVING MATCHED MELTING TEMPERATURES

    (iii) NUMBER OF SEQUENCES: 65

    (iv) COMPUTER READABLE FORM:

        (A) MEDIUM TYPE: Diskette, 3.5 inch, 1.44 MB storage (IBM)

        (B) COMPUTER: IBM PS/2

        (C) OPERATING SYSTEM: MS-DOS Version 3.3

        (D) SOFTWARE: PC-8 (Word for Windows)

    (vi) CURRENT APPLICATION DATA:

        (A) APPLICATION NUMBER: To Be Assigned

(2) INFORMATION FOR SEQ ID:NO:1

    (i) SEQUENCE CHARACTERISTICS:

        (A) LENGTH: 28 nucleotides

        (B) TYPE: Nucleic acid

        (C) STRANDEDNESS: Single

        (D) TOPOLOGY: Linear

    (xi) SEQUENCE DESCRIPTION: SEQ ID:NO:1

        AGTGGGGGGA CATCAAGCAG CCATGCAA   28

(3)    INFORMATION FOR SEQ ID:NO:2

    (i)    SEQUENCE CHARACTERISTICS:

        (A)    LENGTH:   28 nucleotides

        (B)    TYPE:   Nucleic acid

        (C)    STRANDEDNESS:   Single

        (D)    TOPOLOGY:   Linear

    (xi) SEQUENCE DESCRIPTION:   SEQ ID:NO:2


        TTCCTGCTAT GTCACTTCCC CTTGGTTC   28


(4)    INFORMATION FOR SEQ ID:NO:3

    (i)    SEQUENCE CHARACTERISTICS:

        (A)    LENGTH:   28 nucleotides

        (B)    TYPE:   Nucleic acid

        (C)    STRANDEDNESS:   Single

        (D)    TOPOLOGY:   Linear

    (xi) SEQUENCE DESCRIPTION:   SEQ ID:NO:3


        TAGCACCCAC CAGGGCAAAG AGAAGAGT   28


(5)    INFORMATION FOR SEQ ID:NO:4

    (i)    SEQUENCE CHARACTERISTICS:

        (A)    LENGTH:   28 nucleotides

        (B)    TYPE:   Nucleic acid

        (C)    STRANDEDNESS:   Single

        (D)    TOPOLOGY:   Linear

    (xi) SEQUENCE DESCRIPTION:   SEQ ID:NO:4


        AGATGCTGTT GCGCCTCAAT AGCCCTCA   28

(6)   INFORMATION FOR SEQ ID:NO:5
      (i)   SEQUENCE CHARACTERISTICS:
            (A)   LENGTH:  26 nucleotides
            (B)   TYPE:  Nucleic acid
            (C)   STRANDEDNESS:  Single
            (D)   TOPOLOGY:  Linear
      (xi) SEQUENCE DESCRIPTION:  SEQ ID:NO:5


            CTTGGTTCTC TCATCTGGCC TGGTGC   26


(7)   INFORMATION FOR SEQ ID:NO:6
      (i)   SEQUENCE CHARACTERISTICS:
            (A)   LENGTH:  28 nucleotides
            (B)   TYPE:  Nucleic acid
            (C)   STRANDEDNESS:  Single
            (D)   TOPOLOGY:  Linear
      (xi) SEQUENCE DESCRIPTION:  SEQ ID:NO:6


            GAGACCATCA ATGAGGAAGC TGCAGAAT   28


(8)   INFORMATION FOR SEQ ID:NO:7
      (i)   SEQUENCE CHARACTERISTICS:
            (A)   LENGTH:  28 nucleotides
            (B)   TYPE:  Nucleic acid
            (C)   STRANDEDNESS:  Single
            (D)   TOPOLOGY:  Linear
      (xi) SEQUENCE DESCRIPTION:  SEQ ID:NO:7


            GTGCAGCAGC AGAACAATTT GCTGAGGG   28

(9)    INFORMATION FOR SEQ ID:NO:8
       (i)    SEQUENCE CHARACTERISTICS:
              (A)    LENGTH:  30 nucleotidses
              (B)    TYPE:  Nucleic acid
              (C)    STRANDEDNESS:  Single
              (D)    TOPOLOGY:  Linear
       (xi) SEQUENCE DESCRIPTION:   SEQ ID:NO:8


          GGTGTCACCC CCAGAGTCCC CTGTACCCGC   30


(10)  INFORMATION FOR SEQ ID:NO:9
       (i)    SEQUENCE CHARACTERISTICS:
              (A)    LENGTH:  41 nucleotides
              (B)    TYPE:  Nucleic acid
              (C)    STRANDEDNESS:  Single
              (D)    TOPOLOGY:  Linear
       (xi) SEQUENCE DESCRIPTION:   SEQ ID:NO:9


       ATCCTGGGAT TAAATAAAAT AGTAAGAATG TATAGCCCTA C   41


1)    INFORMATION FOR SEQ ID:NO:10
       (i)    SEQUENCE CHARACTERISTICS:
              (A)    LENGTH:  25 nucleotides
              (B)    TYPE:  Nucleic acid
              (C)    STRANDEDNESS:  Single
              (D)    TOPOLOGY:  Linear
       (xi) SEQUENCE DESCRIPTION:   SEQ ID:NO:10


              TGCACTGCCA GGTGCTTCGG CTCAT    25

(12) INFORMATION FOR SEQ ID:NO:11

    (i)   SEQUENCE CHARACTERISTICS:

        (A)   LENGTH:  25 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:11

          CACCACGCAG CGGCCCTTGA TGTTT    25

(13) INFORMATION FOR SEQ ID:NO:12

    (i)   SEQUENCE CHARACTERISTICS:

        (A)   LENGTH:  30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:12

          GAACCGAGGG CCGGCTCACC TCTATGTTGG  30

(14) INFORMATION FOR SEQ ID:NO:13

    (i)   SEQUENCE CHARACTERISTICS:

        (A)   LENGTH:  30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:13

          CCTGCTATGT CACTTCCCCT TGGTTCTCTC  30

(15) INFORMATION FOR SEQ ID:NO:14

    (i)   SEQUENCE CHARACTERISTICS:

        (A)   LENGTH:  27 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:14

        AAGTAGACCA ACAGCACCAC CTAGCGG    27

(16) INFORMATION FOR SEQ ID:NO:15

    (i)   SEQUENCE CHARACTERISTICS:

        (A)   LENGTH:  29 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:15

        GCAGCCTTCT GAGAGTGCCT GAAATCCTG    29

(17) INFORMATION FOR SEQ ID:NO:16

    (i)   SEQUENCE CHARACTERISTICS:

        (A)   LENGTH:  28 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:16

        GGGATAGTGC AGCAACAGCA ACAGCTGT    28

(18) INFORMATION FOR SEQ ID:NO:17

    (i) SEQUENCE CHARACTERISTICS:

        (A) LENGTH: 29 nucleotides

        (B) TYPE: Nucleic acid

        (C) STRANDEDNESS: Single

        (D) TOPOLOGY: Linear

    (xi) SEQUENCE DESCRIPTION: SEQ ID:NO:17


GTGGCAGACT TGTCTAAACG CACATCCCC 29


(19) INFORMATION FOR SEQ ID:NO:18

    (i) SEQUENCE CHARACTERISTICS:

        (A) LENGTH: 26 nucleotides

        (B) TYPE: Nucleic acid

        (C) STRANDEDNESS: Single

        (D) TOPOLOGY: Linear

    (xi) SEQUENCE DESCRIPTION: SEQ ID:NO:18


GAGGAAAAGA AGTTCGGGGC AGAAGT 26


(20) INFORMATION FOR SEQ ID:NO:19

    (i) SEQUENCE CHARACTERISTICS:

        (A) LENGTH: 28 nucleotides

        (B) TYPE: Nucleic acid

        (C) STRANDEDNESS: Single

        (D) TOPOLOGY: Linear

    (xi) SEQUENCE DESCRIPTION: SEQ ID:NO:19


CAACAAGAAA TGTTGCGACT GACCGTCT 28

(21) INFORMATION FOR SEQ ID:NO:20

    (i)   SEQUENCE CHARACTERISTICS:

        (A)   LENGTH:  37 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:20

CGTCGTCGTA TAATCCACCT ATCCCAGTAG GAGAAAT   37

(22) INFORMATION FOR SEQ ID:NO:21

    (i)   SEQUENCE CHARACTERISTICS:

        (A)   LENGTH:  37 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:21

CGTCGTCGTT TTGGTCCTTG TCTTATGTCC AGAATGC   37

(23) INFORMATION FOR SEQ ID:NO:22

    (i)   SEQUENCE CHARACTERISTICS:

        (A)   LENGTH: 28 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:22

ATAATCCACC TATCCCAGTA GGAGAAAT ` 28

(24) INFORMATION FOR SEQ ID:NO:23

    (i)   SEQUENCE CHARACTERISTICS:

        (A)   LENGTH:  28 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:23

TTTGGTCCTT GTCTTATGTC CAGAATGC    28

(25) INFORMATION FOR SEQ ID:NO:24

    (i)   SEQUENCE CHARACTERISTICS:

        (A)   LENGTH:  40 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:24

GATGGATGAC AAATAATCCA CCTATCCCAG TAGGAGAAAT    40

(26) INFORMATION FOR SEQ ID:NO:25

    (i)   SEQUENCE CHARACTERISTICS:

        (A)   LENGTH:  40 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:25

CTAAAGGGTT CCTTTGGTCC TTGTCTTATG TCCAGAATGC    40

(27)  INFORMATION FOR SEQ ID:NO:26

    (i)   SEQUENCE CHARACTERISTICS:

        (A)   LENGTH:  29 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:26

        GAGATGGGAA TCCATATGCT GTATGTGAT    29

(28)  INFORMATION FOR SEQ ID:NO:27

    (i)   SEQUENCE CHARACTERISTICS:

        (A)   LENGTH:  29 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:27

        GGACACAGTG GCTTTTGACA GTTAATACA    29

(29)  INFORMATION FOR SEQ ID:NO:28

    (i)   SEQUENCE CHARACTERISTICS:

        (A)   LENGTH:  25 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:28

        GATGGTCCAG CTGGACAAGC AGAAC    25

(30)  INFORMATION FOR SEQ ID:NO:29

     (i)   SEQUENCE CHARACTERISTICS:

         (A)   LENGTH:  26 nucleotides

         (B)   TYPE:  Nucleic acid

         (C)   STRANDEDNESS:  Single

         (D)   TOPOLOGY:  Linear

     (xi) SEQUENCE DESCRIPTION:  SEQ ID:NO:29


         CCTAGTGTGC CCATTAACAG GTCTTC    26


(31)  INFORMATION FOR SEQ ID:NO:30

     (i)   SEQUENCE CHARACTERISTICS:

         (A)   LENGTH:  29 nucleotides

         (B)   TYPE:  Nucleic acid

         (C)   STRANDEDNESS:  Single

         (D)   TOPOLOGY:  Linear

     (xi) SEQUENCE DESCRIPTION:  SEQ ID:NO:30


         GACACAGAAA ATGCTAGTGC TTATGCAGC    29


(32)  INFORMATION FOR SEQ ID:NO:31

     (i)   SEQUENCE CHARACTERISTICS:

         (A)   LENGTH:  29 nucleotides

         (B)   TYPE:  Nucleic acid

         (C)   STRANDEDNESS:  Single

         (D)   TOPOLOGY:  Linear

     (xi) SEQUENCE DESCRIPTION:  SEQ ID:NO:31


         GGTGGACAAT CACCTGGATT TACTGCAAC    29

(33) INFORMATION FOR SEQ ID:NO:32

    (i)   SEQUENCE CHARACTERISTICS:

        (A)   LENGTH:  27 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:32

        CCTGATCTGT GCACGGAACT GAACACT    27

(34) INFORMATION FOR SEQ ID:NO:33

    (i)   SEQUENCE CHARACTERISTICS:

        (A)   LENGTH: 32 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:33

        CCCAGTGTTA GTTAGTTTTT CCAATGTGTC TG    32

(35) INFORMATION FOR SEQ ID:NO:34

    (i)   SEQUENCE CHARACTERISTICS:

        (A)   LENGTH:  27 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:34

        TGCCTGCGGT GCCAGAAACC GTTGAAT    27

(36) INFORMATION FOR SEQ ID:NO:35

    (i)   SEQUENCE CHARACTERISTICS:

        (A)   LENGTH: 26 nucleotides

        (B)   TYPE:  Nucleic acid

        (C)   STRANDEDNESS:  Single

        (D)   TOPOLOGY:  Linear

  (xi) SEQUENCE DESCRIPTION:  SEQ ID:NO:35


      TGCTCGGTTG CAGCACGAAT GCCACT    26


(37) INFORMATION FOR SEQ ID:NO:36

    (i)   SEQUENCE CHARACTERISTICS:

        (A)   LENGTH:  28 nucleotides

        (B)   TYPE:  Nucleic acid

        (C)   STRANDEDNESS:  Single

        (D)   TOPOLOGY:  Linear

  (xi) SEQUENCE DESCRIPTION:  SEQ ID:NO:36


      GAGCCGAACC ACAACGTCAC ACAATGTT    28


(38) INFORMATION FOR SEQ ID:NO:37

    (i)   SEQUENCE CHARACTERISTICS:

        (A)   LENGTH:  29 nucleotides

        (B)   TYPE:  Nucleic acid

        (C)   STRANDEDNESS:  Single

        (D)   TOPOLOGY:  Linear

  (xi) SEQUENCE DESCRIPTION:  SEQ ID:NO:37


      GGACACACAA AGGACAGGGT GTTCAGAAA    29

(39) INFORMATION FOR SEQ ID:NO:38

    (i)    SEQUENCE CHARACTERISTICS:

        (A)    LENGTH:  27 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:38

CATTCCCACT GACTTTCTGA CGCACGT    27

(40) INFORMATION FOR SEQ ID:NO:39

    (i)    SEQUENCE CHARACTERISTICS:

        (A)    LENGTH:  24 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:39

GCGACTCAGA GGAAGAAAAC GATG    24

(41) INFORMATION FOR SEQ ID:NO:40

    (i)    SEQUENCE CHARACTERISTICS:

        (A)    LENGTH:  25 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:40

GAGATCGAGC TGGAGGATCC GTACG    25

(42) INFORMATION FOR SEQ ID:NO:41

    (i)    SEQUENCE CHARACTERISTICS:

        (A)    LENGTH:  25 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:41

          AGCTGCAGCC CAAAGGTGTT GGACT    25

(43) INFORMATION FOR SEQ ID:NO:42

    (i)    SEQUENCE CHARACTERISTICS:

        (A)    LENGTH:  21 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:42

          TCAGCCGCGT CCACGCCGCG A    21

(44) INFORMATION FOR SEQ ID:NO:43

    (i)    SEQUENCE CHARACTERISTICS:

        (A)    LENGTH:  20 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:43

          CCTGCGAGCG TAGGCGTCGG    20

(45) INFORMATION FOR SEQ ID:NO:44

    (i)   SEQUENCE CHARACTERISTICS:

        (A)   LENGTH:  20 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:44


        GAGATCGCCA CCTTCGGCAA    20


(46) INFORMATION FOR SEQ ID:NO:45

    (i)   SEQUENCE CHARACTERISTICS:

        (A)   LENGTH:  20 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:45


        GAGCAGTTCG GTGGCGTTCA    20


(47) INFORMATION FOR SEQ ID:NO:46

    (i)   SEQUENCE CHARACTERISTICS:

        (A)   LENGTH:  25 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:46


        GAGGCTATTG TAGCCTACAC TTTGG  25

(48)  INFORMATION FOR SEQ ID:NO:47

    (i)   SEQUENCE CHARACTERISTICS:

        (A)   LENGTH:  25 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:47

        CAGCACCATC CTCCTCTTCC TCTGG   25

(49)  INFORMATION FOR SEQ ID:NO:48

    (i)   SEQUENCE CHARACTERISTICS:

        (A)   LENGTH:  24 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:48

        TGAGGTCGTG GAACTTGATG GCGT   24

(50)  INFORMATION FOR SEQ ID:NO:49

    (i)   SEQUENCE CHARACTERISTICS:

        (A)   LENGTH:  30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:49

        GACACAGTGT CCTCCCGCTC CTCCTGAGCA   30

(51) INFORMATION FOR SEQ ID:NO:50

    (i)    SEQUENCE CHARACTERISTICS:

        (A)    LENGTH:  30 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:50


        GTGGAAGGCG GCTCGCTGGA AGCCGGTCGT  30


(52) INFORMATION FOR SEQ ID:NO:51

    (i)    SEQUENCE CHARACTERISTICS:

        (A)    LENGTH: 33 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:51


        GGAACAACAT TAGAACAGCA ATACAACAAA CCG    33


(53) INFORMATION FOR SEQ ID:NO:52

    (i)    SEQUENCE CHARACTERISTICS:

        (A)    LENGTH:  32 nucleotides

        (B)    TYPE:  Nucleic acid

        (C)    STRANDEDNESS:  Single

        (D)    TOPOLOGY:  Linear

    (xi) SEQUENCE DESCRIPTION:  SEQ ID:NO:52


        AATATTGTAA CCTTTTGTTG CAAGTGTGAC TC    32

(54) INFORMATION FOR SEQ ID:NO:53

    (i)   SEQUENCE CHARACTERISTICS:

        (A)   LENGTH:  30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:53

        CCTATAGGTG GTTTGCAACC AATTAAACAC    30

(55) INFORMATION FOR SEQ ID:NO:54

    (i)   SEQUENCE CHARACTERISTICS:

        (A)   LENGTH:  35 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:54

        GAGGTATTTG AATTTGCATT TAAAGATTTA TTTGT  35

(56) INFORMATION FOR SEQ ID:NO:55

    (i)   SEQUENCE CHARACTERISTICS:

        (A)   LENGTH:  27 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:55

        GCAAGACAGT ATTGGAACTT ACAGAGG    27

(57) INFORMATION FOR SEQ ID:NO:56

    (i)   SEQUENCE CHARACTERISTICS:

        (A)   LENGTH: 27 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:56


        GTGTTGTAAG TGTGAAGCCA GATTTGA    27


(58) INFORMATION FOR SEQ ID:NO:57

    (i)   SEQUENCE CHARACTERISTICS:

        (A)   LENGTH:  30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:57


        GAGCAGATTG CGGCCACCGC AGCGATTTCG    30


(59) INFORMATION FOR SEQ ID:NO:58

    (i)   SEQUENCE CHARACTERISTICS:

        (A)   LENGTH:  20 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:58


        CTCGTCCAGC GCCGCTTCGG    20

(60) INFORMATION FOR SEQ ID:NO:59

    (i)   SEQUENCE CHARACTERISTICS:

        (A)   LENGTH: 20 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:59


          TGGATCTCGT TGTTCGGGTC    20


(61) INFORMATION FOR SEQ ID:NO:60

    (i)   SEQUENCE CHARACTERISTICS:

        (A)   LENGTH: 30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:60


        GACCAGATCG CTGCCACCGC GGCCATCTCC    30


(62) INFORMATION FOR SEQ ID:NO:61

    (i)   SEQUENCE CHARACTERISTICS:

        (A)   LENGTH: 30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:61


        GAGCAGATCG CTGCCACCGC CGGTATCTCC    30

(63) INFORMATION FOR SEQ ID:NO:62

    (i)   SEQUENCE CHARACTERISTICS:

        (A)   LENGTH:  30 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:62

        GGTCATCGCC GTAGTAGATG CGTAAGGCCT    30

(64) INFORMATION FOR SEQ ID:NO:63

    (i)   SEQUENCE CHARACTERISTICS:

        (A)   LENGTH: 25 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:63

        CCGGGAGATG GGGGAGGCTA ACTGA    25

(65) INFORMATION FOR SEQ ID:NO:64

    (i)   SEQUENCE CHARACTERISTICS:

        (A)   LENGTH: 25 nucleotides

        (B)   TYPE:  Nucleic acid

        (C)   STRANDEDNESS:  Single

        (D)   TOPOLOGY:  Linear

    (xi) SEQUENCE DESCRIPTION:  SEQ ID:NO:64

        GGGGTGGGGA AAAGGAAGAA ACGCG    25

41

```
(66)  INFORMATION FOR SEQ ID:NO:65
          (i)   SEQUENCE CHARACTERISTICS:
                (A)   LENGTH: 33 nucleotides
                (B)   TYPE:  Nucleic acid
                (C)   STRANDEDNESS:  Single
                (D)   TOPOLOGY:  Linear
          (xi) SEQUENCE DESCRIPTION:  SEQ ID:NO:65


          AAAGACAGAA  TAAAACGCAC  GGGTGTTGGG  TCG      33
```

## Claims

1. An aqueous composition buffered to a pH of from 7 to 9, and comprising:

   a) first and second primers which are specific to and hybridizable with, respectively, first and second nucleic acid sequences which are in opposing strands of a first target DNA and which are separated from each other along the opposing strands by from 90 to 400 nucleotides, and

   b) third and fourth primers which are specific to and hybridizable with, respectively, third and fourth nucleic acid sequences which are in opposing strands of a second target DNA which is the same as or different from the first target DNA, the third and fourth nucleic acid sequences being different from the first and second nucleic acid sequences and being separated from each other along the opposing strands by from 90 to 400 nucleotides,

   the composition characterized wherein each of the first, second, third and fourth primers has a $T_m$ within the range of from 65 to 74 $^\circ$C, all of the primer $T_m$'s being within 5 $^\circ$C of each other, the first and second primers have nucleotide lengths which differ from each other by no more than 5 nucleotides, and the third and fourth primers have nucleotide lengths which differ from each other by no more than 5 nucleotides.

2. The composition as claimed in claim 1 wherein each of the primers is present at the same amount at a concentration of at least 0.075 $\mu$molar, and the composition further comprises

   a thermostable DNA polymerase present at from 10 to 50 units/100 $\mu$l,

   a DNA polymerase cofactor present at from 2 to 15 $\mu$molar, and

   a dNTP present at from 0.25 to 3.5 $\mu$molar.

3. The composition as claimed in either of claims 1 and 2 wherein each of the first, second, third and fourth primers has from 20 to 40 nucleotides, and a $T_m$ within the range of from 67 to 74 $^\circ$C, theprimer $T_m$'s being within 2 $^\circ$C of each other.

4. The composition as claimed in any of claims 1 to 3 wherein the $T_m$ values are calculated using the formula:

   $$T_m (^\circ C) = 67.5 + 0.34(\%G + C) - 395/N$$

   wherein G and C represent the number of guanine and cytosine nucleotides, respectively, and N represents the total number of nucleotides.

5. The composition as claimed in any of claims 1 to 4 wherein one or both of the first and second primers, and one or both of the third and fourth primers, are labeled with the same or different specific binding moiety.

6. A diagnostic test kit for the amplification of first and second target DNA's comprising, in separate packaging:

a) an aqueous composition as claimed in any of claims 1 to 5, and

b) at least one additional PCR reagent.

7. The test kit as claimed in claim 6 further comprising

a first capture reagent comprising a water-insoluble support to which is covalently attached a first capture probe which is specific to a nucleic acid sequence of a strand of the first target DNA, the first capture probe having from 10 to 40 nucleotides and a $T_m$ greater than 50°C, and being hybridizable with the nucleic acid sequence of the first target DNA strand at a temperature in the range of from 40 to 55°C, and

a second capture reagent comprising a water-insoluble support to which is covalently attached a second capture probe which is specific to a nucleic acid sequence of a strand of the second target DNA, the second capture probe having from 10 to 40 nucleotides and a $T_m$ greater than 50°C, and being hybridizable with the nucleic acid sequence of the second target DNA strand at a temperature in the range of from 40 to 55°C.

8. A method for the simultaneous amplification and detection of a first target DNA and a second target DNA comprising:

A) simultaneously subjecting the denatured opposing strands of a first target DNA and the denatured opposing strands of a second target DNA to polymerase chain reaction in the presence of:

i) the aqueous composition as claimed in any of claims 1 to 5, and

ii) the additional PCR reagents: a thermostable DNA polymerase, a DNA polymerase cofactor and dNTP's, any or all of the additional PCR reagents being in the same or a different composition as defined in i),

to simultaneously amplify the opposing first target DNA strands and the opposing second target DNA strands, and

B) simultaneously detecting at least one of the amplified first target DNA strands and at least one of the amplified second target DNA strands as a simultaneous determination of the presence of the first and second target DNA's.

9. The method as claimed in claim 8 wherein the labeled primers are labeled with biotin, and detection of the resulting biotinylated amplified DNA strands for either target DNA is achieved by reacting the biotinylated amplified DNA strands with an avidin-enzyme conjugate, followed by reaction of the enzyme with a substrate reagent to produce a detectable colorimetric or chemiluminescent signal.

10. The method as claimed in either of claims 8 or 9 wherein, in each PCR cycle, priming and primer extension are carried out at the same temperature within the range of from 62 to 75°C.

11. The method as claimed in any of claims 8 to 10 wherein one of the amplified first target DNA strands is captured with a first capture reagent comprising a water-insoluble support to which is covalently attached a first capture probe which is specific to a nucleic acid sequence of the first target DNA strand, the first capture probe having from 10 to 40 nucleotides and a $T_m$ greater than 50°C, and is hybridizable with the nucleic acid sequence of the first target DNA strand at a temperature in the range of from 40 to 55°C, and

one of the amplified second target DNA strands is captured with a second capture reagent comprising a second capture probe specific to a nucleic acid sequence of the second target DNA strand, the second capture probe having from 10 to 40 nucleotides and a $T_m$ greater than 50°C, and is hybridizable with the nucleic acid sequence of the second target DNA strand at a temperature in the range of from 40 to 55°C,

the first and second capture probes having $T_m$'s which differ by no more than 15°C.

12. The method as claimed in claim 11 wherein the water-insoluble support for each capture reagent is a polymeric or magnetic particle having a diameter in the range of from 0.001 to 10 micrometers, and each of the capture probes has a $T_m$ greater than 55°C.

13. The method as claimed in either of claims 11 or 12 wherein the first and second capture reagents are disposed in distinct regions on a water-insoluble substrate of a test device.

**14.** A diagnostic element comprising a water-insoluble, heat or ultrasonic sealable support, having disposed thereon in distinct regions thereof, a plurality of capture reagents,

the element characterized wherein each of the capture reagents has a capture probe specific for and hybridizable with a distinct target DNA associated with an infectious agent at a temperature of from 40 to 55°C, each of the capture probes has from 10 to 40 nucleotides and a $T_m$ greater than 50°C, and the $T_m$'s of all capture probes differ by no more than 15°C.

**15.** An oligonucleotide having the sequence:

```
GAGATGGGAA TCCATATGCT GTATGTGAT,
GGACACAGTG GCTTTTGACA GTTAATACA,
 GATGGTCCAG CTGGACAAGC AGAAC,
  CCTAGTGTGC CCATTAACAG GTCTTC,
GACACAGAAA ATGCTAGTGC TTATGCAGC,
GGTGGACAAT CACCTGGATT TACTGCAAC,
  CCTGATCTGT GCACGGAACT GAACACT,
CCCAGTGTTA GTTAGTTTTT CCAATGTGTC TG,
  TGCCTGCGGT GCCAGAAACC GTTGAAT,
   TGCTCGGTTG CAGCACGAAT GGCACT,
 GAGCCGAACC ACAACGTCAC ACAATGTT,
 GGACACACAA AGGACAGGGT GTTCAGAAA,
  GCGACTCAGA GGAAGAAAAC GATG,
  GAGATCGAGC TGGAGGATCC GTACG,
  AGCTGCAGCC CAAAGGTGTT GGACT,
GGAACAACAT TAGAACAGCA ATACAACAAA CCG,
AATATTGTAA CCTTTTGTTG CAAGTGTGAC TC,
 CCTATAGGTG GTTTGCAACC AATTAAACAC,
GAGGTATTTG AATTTGCATT TAAAGATTTA TTTGT,
 GCAAGACAGT ATTGGAACTT ACAGAGG,
 GTGTTGTAAG TGTGAAGCCA GATTTGA,
GAGCAGATTG CGGCCACCGC AGCGATTTCG,
 CCGGGAGATG GGGGAGGCTA ACTGA,
 GGGGTGGGGA AAAGGAAGAA ACGCG, or
AAAGACAGAA TAAAACGCAC GGGTGTTGGG TCG.
```

FIG.2

FIG.1

EP 0 630 973 A2

FIG. 3

FIG. 4

FIG. 6

FIG. 5

EP 0 630 973 A2

FIG. 7

FIG. 8

FIG. 9

FIG. 10